# EUROPÄISCHE PATENTSCHRIFT

(11) **EP 4 493 263 B1**
(45) Veröffentlichungstag und Bekanntmachung des Hinweises auf die Patenterteilung: **25.02.2026**
(21) Anmeldenummer: 22712642.2
(22) Anmeldetag: 18.03.2022
(51) Int. Cl.: A61M 60/812, A61M 60/13, A61M 60/892

(54) **BLUTPUMPE ZUR UNTERSTÜTZUNG DER LEISTUNG EINES HERZENS**
BLOOD PUMP FOR SUPPORTING CARDIAC PERFORMANCE
POMPE À SANG DESTINÉE À ASSISTER L'ACTIVITÉ CARDIAQUE

(30) Priorität: 14.03.2022 AT 652022
(43) Veröffentlichungstag der Anmeldung: 22.01.2025
(73) Patentinhaber: MOHL, Werner, 2571 Altenmarkt/Thennenberg (AT)
(72) Erfinder: SIEKMEYER, Gerd, 24159 Kiel (DE); MOHL, Werner, 2571 Altenmarkt-Thennenberg (AT)
(74) Vertreter: SONN Patentanwälte GmbH & Co KG
(86) Internationale Anmeldenummer: PCT/IB2022/052465
(87) Internationale Veröffentlichungsnummer: WO 2023/175380

(56) Entgegenhaltungen:
- US-A1- 2002 123 661
- US-A1- 2021 199 120

## Beschreibung

Die Erfindung betrifft eine Blutpumpe zur Unterstützung der Leistung eines Herzens.

Eine Blutpumpe mit Bezug zur Erfindung gemäß der vorliegenden Anmeldung ist beispielsweise in US 2021/199120 A1 offenbart.

Blutpumpen zur Unterstützung der Leistung eines Herzens werden zur temporären mechanischen Unterstützung der Herztätigkeit bei akuten kardiologischen Zuständen, wie z.B. Herzinsuffizienz, Herzinfarkt oder chronischem Herzversagen eingesetzt und dienen der Vermeidung eines kardiogenen Schocks bzw. der hämodynamischen Stabilisierung im kardiogenen Schock.

Zur Unterstützung der Herztätigkeit während einer perkutanen Koronarintervention wurden bereits intraaortale Ballonpumpen vorgeschlagen. Intraaortale Ballonpumpen bestehen aus einem Ballonkatheter, der unterhalb der linken Arteria subclavia und oberhalb des Abganges der Nierenarterien in der Aorta descendens platziert wird, sowie aus einer extrakorporalen Pumpe mit einem Steuergerät. Die Pumpe füllt den Ballon des Ballonkatheters in der Diastole und saugt den Ballon unmittelbar vor Beginn der Systole vollständig leer.

Intraaortale Ballonpumpen dienen der intraaortalen Gegenpulsation. Die intraaortale Gegenpulsation wird durch die Füllung und Entleerung des Ballons und die daraus resultierende Änderung der Druckverhältnisse innerhalb der Aorta erreicht. Im gefüllten Zustand verhindert der Ballon den Blutfluss in Richtung der unteren Körperhälfte, wodurch der diastolische Aortendruck ansteigt und der Blutfluss in Richtung der oberen Körperhälfte und des Herzens erhöht wird (sog. diastolische Augmentation). Die diastolische Augmentation führt zu einer Verbesserung der koronaren und zerebralen Durchblutung während der Diastole. Die aktive Entleerung des Ballons unmittelbar vor der Systole gibt den Blutfluss in Richtung der unteren Körperhälfte frei, wodurch der enddiastolische und systolische Aortendruck gesenkt wird. Der gesenkte Druck in der Aorta führt während der Systole zu einer Erniedrigung des intraaortalen Widerstandes, einer Senkung der linksventrikulären Nachlast und einer damit einhergehenden Steigerung des Herzzeitvolumens, sowie einer Entlastung des Myokards.

Der Nachteil bei intraaortalen Ballonpumpen gemäß dem Stand der Technik liegt darin, dass die intraaortale Gegenpulsation lediglich eine relativ geringe Erhöhung der Herzzeitvolumens bewirkt.

Zur Unterstützung der Herztätigkeit während einer perkutanen Koronarintervention wurden auch kleine Axialpumpen vorgeschlagen (sog. perkutane ventrikuläre Herzunterstützungssysteme, PVAD), die über ein arterielles Gefäß bis zum Herz vorgeschoben werden. Die Pumpe wird derart angeordnet, dass das Blut im linken Ventrikel von der Pumpe aufgenommen wird und in die Aorta ascendens gepumpt wird. Die Pumpe wird kontinuierlich betrieben, d.h. unabhängig vom Herzzyklus, was zu einer volumenabhängigen Entlastung des linken Ventrikels führt. Mit zunehmender Pumpleistung wird der linke Ventrikel zunehmend entlastet, was zu einer Verringerung des linksventrikulären enddiastolischen Drucks, einer Abnahme der LV-Arbeit und des myokardialen Sauerstoffbedarfs führt. Außerdem führt der höhere Grad der Entlastung zu einer verstärkten Dissoziation von LV-Spitzendruck und Aortendruck, die als ventrikulo-arterielle Entkopplung bezeichnet wird.

Anders als intraaortale Ballonpumpen sind solche Axialpumpen jedoch nicht ohne weiteres geeignet, die koronare Durchblutung zu verbessern.

Eine besondere Herausforderung stellt die perkutane Koronarintervention bei Risikopatienten, sog. CHIP ("complex high-risk and indicated patients"), dar. Solche Risikopatienten werden durch eine Reihe von Merkmalen definiert, darunter eine komplexe koronare Herzkrankheit (KHK, Mehrgefäß- oder Linksherzerkrankung und anatomisch komplexe Koronarläsionen), der hämodynamische Status (stark eingeschränkte LV-Funktion) und klinische Begleiterkrankungen wie fortgeschrittenes Alter, Diabetes, periphere Gefäßerkrankungen, Herzinsuffizienz, akute Koronarsyndrome oder frühere Herzoperationen. Insbesondere handelt es sich hierbei um eine Patientengruppe, bei der die Durchblutung des Herzens durch die Intervention zeitweise beeinträchtigt wird und im Fall einer Komplikation sogar unterbrochen werden kann.

Die vorliegende Erfindung zielt daher darauf ab, die Vorteile einer intraaortalen Ballonpumpe mit jenen der Axialpumpe zu verbinden, sodass sowohl die intraaortale Gegenpulsation, als auch eine erhöhte Auswurfleistung erreicht werden kann, um Komplikationen bei der perkutanen Koronarintervention von Hochrisikopatienten zu verringern.

Zur Lösung dieser Aufgabe umfasst die erfindungsgemäße Blutpumpe zur Unterstützung der Leistung eines Herzens ein stentartiges Gehäuse, das ein erstes axiale Ende und ein zweites axiales Ende aufweist, einen im Gehäuse drehbar gelagerten Pumpenrotor, insbesondere Impeller, und eine flexible Antriebswelle, die mit einem Motor koppelbar ist, um den Pumpenrotor zur Drehbewegung anzutreiben, wodurch Blut auf einer Ansaugseite des Pumpenrotors durch einen Ansaugschlauch der Blutpumpe eingesaugt und auf einer Ausstoßseite des Pumpenrotors ausgeworfen wird, wobei die Blutpumpe ein stromabwärts des Pumpenrotors angeordnetes Strömungsleitmittel aufweist, um das vom Pumpenrotor geförderte Blut unter Beibehaltung der Drehrichtung des Pumpenrotors wahlweise zum ersten oder zum zweiten axialen Ende des Gehäuses zu leiten.

Die erfindungsgemäße Blutpumpe wird beispielsweise in der Aorta descendens platziert, wobei die Ansaugseite, d.h. das erste axiale Ende des Gehäuses in unmittelbarer Nähe der linken Arteria subclavia und die Ausstoßseite, d.h. das zweite axiale Ende des Gehäuses distal der linken Arteria subclavia angeordnet ist. Der Ansaugschlauch der Pumpe ragt hierbei über das erste axiale Ende des Gehäuses vor und reicht zumindest bis in den Aortenbogen, wobei die Ansaugöffnung des Ansaugschlauches in der Aorta ascendens oder im linken Ventrikel angeordnet ist.

Alternativ kann die erfindungsgemäße Blutpumpe beispielweise auch derart angeordnet sein, dass die Ansaugseite der Pumpe im linken Ventrikel und die Ausstoßseite der Pumpe in der Aorta ascendens angeordnet ist.

Das Vorsehen eines Strömungsleitmittels, welches das vom Pumpenrotor geförderte Blut unter Beibehaltung der Drehrichtung des Pumpenrotors wahlweise zum ersten oder zum zweiten axialen Ende des Gehäuses leitet, ermöglicht einerseits bei entsprechendem Wechsel der Ausstoßrichtung den Effekt einer intraaortalen Gegenpulsation und andererseits auf Grund der Pumpwirkung eine Erhöhung des Herzzeitvolumens. Dadurch werden die Vorteile einer intraaortalen Ballonpumpe mit jenen der Axialpumpe kombiniert.

Eine konstruktiv vorteilhafte Ausgestaltung der Blutpumpte sieht vor, dass der Impeller einen inneren Impellerkörper aufweist, der eine Mehrzahl von Impellerschaufeln trägt und von einem im Wesentlichen konischen Impellermantel derart umgeben ist, dass zwischen dem Impellermantel und dem Impellerkörper ein ringförmiger, sich in Strömungsrichtung von der Ansaugseite zu der Ausstoßseite radial nach außen erweiternder Strömungskanal ausgebildet wird, in dem die Impellerschaufeln angeordnet sind, wobei ansaugseitig der Ansaugschlauch mit dem Impellermantel verbunden ist und der Strömungskanal ausstoßseitig eine ringförmige Öffnung aufweist, deren äußerer Rand von dem Impellermantel und deren innerer Rand von dem Impellerkörper gebildet ist, und wobei das Gehäuse das nach innen ragende Strömungsleitmittel aufweist, das wahlweise mit dem inneren oder dem äußeren Rand der ringförmigen Öffnung zusammenwirkt, um das aus dem Strömungskanal des Impellers austretende Blut entweder zum ersten oder zum zweiten axialen Ende des Gehäuses zu leiten.

Bei einer Drehung des Impellers der erfindungsgemäßen Blutpumpe wird das Blut über den Ansaugschlauch aus der linken Herzkammer angesaugt und fließt in den zwischen Impellermantel und Impellerkörper befindlichen Strömungskanal. Der im Strömungskanal angeordnete Impeller stößt das Blut über die ringförmige Öffnung des Strömungskanal aus, wobei ein stromabwärts angeordnetes Strömungsleitmittel erfindungsgemäß derart ausgebildet ist, dass dieses den Blutfluss je nach Einstellung entweder zum ersten oder zum zweiten axialen Ende des Gehäuses leitet.

Ist das Strömungsleitmittel derart angeordnet, dass es mit dem äußeren Rand der ringförmigen Öffnung zusammenwirkt, fließt das Blut in Richtung des zweiten axialen Endes des Gehäuses. Dies führt zu einem Blutfluss in Richtung der unteren Körperhälfte, wodurch der enddiastolische und systolische Aortendruck im Sinne einer intraaortalen Gegenpulsation ähnlich wie bei Verwendung einer intraaortalen Ballonpumpe gesenkt wird. Der gesenkte Druck in der Aorta führt zu einer Senkung der linksventrikulären Nachlast und einer damit einhergehenden Steigerung des Herzzeitvolumens, sowie einer Entlastung des Myokards. Über die Einstellung der Drehzahl des Impellers kann der vorgenannte Effekt an die jeweiligen Bedürfnisse angepasst werden.

Ist das Strömungsleitmittel derart angeordnet, dass es mit dem inneren Rand der ringförmigen Öffnung zusammenwirkt, wird das Blut in die entgegengesetzte Richtung umgeleitet und fließt somit in Richtung des ersten axialen Endes des Gehäuses. Die erfindungsgemäße Pumpe fördert hierbei den Blutfluss aus dem linken Ventrikel in Richtung der oberen Körperhälfte, wodurch der diastolische Aortendruck ansteigt, was wiederum zu einer Verbesserung der zerebralen und koronaren Durchblutung während der Diastole führt. Dieser Effekt kann durch Einstellen der Pumpendrehzahl individuell angepasst werden.

Die erfindungsgemäße Blutpumpe ermöglicht dementsprechend sowohl eine Beschleunigung des aus dem Herzen strömenden Blutes und eine damit einhergehende Steigerung der Auswurfleistung, als auch die intraaortale Gegenpulsation.

Der Antrieb des Pumpenrotors, insbesondere Impellers, erfolgt über eine flexible Antriebswelle, die mit einem Antriebsmotor koppelbar ist. Der Motor wird bevorzugt extrakorporal angeordnet. Wahlweise kann lediglich der die Impellerschaufeln tragende Impellerkörper oder der Impellerkörper gemeinsam mit dem Impellermantel in Drehung versetzt werden.

Das stentartige Gehäuse kann bevorzugt als im Wesentlichen zylindrisches Drahtgeflecht ausgebildet sein, das zumindest in einem axialen Teilbereich beschichtet oder ummantelt sein kann, um in dem betreffenden Teilbereich für Blut undurchlässig zu sein. Die Ummantelung bzw. Beschichtung ist hierbei bevorzugt im Bereich des Impellers vorgesehen, insbesondere vor und nach dem Strömungsleitmittel, um sicherzustellen, dass ein definierter Strömungsquerschnitt für das entweder zum ersten Ende oder zum zweiten Ende des Gehäuses fließenden Blut bereitgestellt wird. Außerhalb des genannten axialen Teilbereichs kann auf eine Beschichtung oder Ummantelung vorzugsweise verzichtet werden, sodass der vom Gehäuse umgebene Bereich der Pumpe mit dem radial außerhalb davon liegenden Volumens des Blutgefäßes, d.h. der Aorta, fluidisch verbunden ist.

Der in den Strömungskanal zwischen dem Impellerkörper und Impellermantel mündende Ansaugschlauch kann ebenfalls als beschichtetes oder ummanteltes Drahtgeflecht ausgebildet sein, um eine ausreichende Flexibilität zu erreichen, welche eine gekrümmte Anordnung im Aortenbogen ermöglicht.

Zur Beschichtung geeignete Materialien umfassen beispielsweise Polyethlen, PC, PET, PTFA und Polyurethan.

Die Impellerschaufeln können bevorzugt von einer Metall-Polymer-Kombination gebildet sein.

Der Impellerkörper kann bevorzugt als Ballon ausgebildet sein, wobei die Impellerschaufeln an der dem Impellermantel zugewandten Seite des Ballons angeordnet sind.

Für die oben beschriebene Veränderung der Strömungsrichtung mit Hilfe des Strömungsleitmittels sind verschiedene konstruktive Ausbildungen denkbar. Gemäß einer ersten Ausführungsform der Erfindung, ist die Blutpumpe bevorzugt derart weitergebildet, dass das Strömungsleitmittel zwischen einer ersten und einer zweiten Position verlagerbar gehalten ist, wobei das Strömungsleitmittel in der ersten Position mit dem äußeren Rand der ringförmigen Öffnung und in der zweiten Position mit dem inneren Rand der ringförmigen Öffnung zusammenwirkt.

Das Strömungsleitmittel kann hierfür beispielsweise als ringförmige bewegliche Klappe ausgebildet sein, deren erstes Ende mittels eines Aktuator-getriebenen Gelenks an der Innenseite des Gehäuses nahe der ringförmigen Öffnung angebracht ist und deren zweites Ende vom Gehäuse in Richtung des Impellers radial nach innen ragt. Je nachdem, welche Flussrichtung des Blutes gewünscht ist, wird die Klappe in Richtung des inneren oder des äußeren Randes der ringförmigen Öffnung bewegt.

Wenn das zweite Ende der Klappe am äußeren Rand der ringförmigen Öffnung, d.h. am Impellermantel anliegt, strömt das Blut durch die ringförmige Öffnung des Strömungskanals in Richtung des zweiten axialen Endes des Gehäuses und gelangt von dort in die untere Körperhälfte. Wenn das zweite Ende der Klappe am inneren Rand der ringförmigen Öffnung, d.h. am Impellerkörper anliegt, trifft das Blut beim Ausströmen aus dem Strömungskanal auf die Klappe auf und wird in Richtung des ersten axialen Endes des Gehäuses geleitet, wodurch ein retrograder Blutfluss in Richtung der oberen Körperhälfte entsteht.

Das Strömungsleitmittel kann bevorzugt von einer ringförmigen, flexiblen Lippe gebildet sein, wobei ein vorzugsweise pneumatisch oder hydraulisch betätigbares Betätigungsglied vorgesehen ist, welches mit dem Strömungsleitmittel zusammenwirkt, um dieses zwischen der ersten und der zweiten Position zu verlagern.

Die ringförmige, flexible Lippe kann beispielsweise als im Längsschnitt des Gehäuses U-förmige Einbuchtung des Gehäuses ausgebildet sein. Bei einer solchen Ausbildung kann das pneumatisch oder hydraulisch betätigbare Betätigungsglied derart in der Einbuchtung aufgenommen sein, dass es die Außenseite des Gehäuses umläuft.

Das pneumatisch oder hydraulisch betätigbare Betätigungsglied kann hierbei von einem ringförmigen Hohlkörper gebildet sein, der mit einer Gaszu- oder Flüssigkeitszu- und -abführungsleitung verbunden ist, um den Füllgrad des Hohlkörpers in gesteuerter Weise zu erhöhen oder zu verringern. Wird Gas oder Flüssigkeit zugeführt und der ringförmige, in der die Außenseite des Gehäuses umfangenden Einbuchtung verlaufende Hohlkörper mit Gas oder Flüssigkeit gefüllt, verringert sich der Innendurchmesser der Gehäuseeinbuchtung, wodurch diese in Kontakt mit dem Impellerkörper gelangt und für eine Umleitung des Blutflusses in Richtung zum ersten Ende des Gehäuses sorgt.

Wird Gas oder Flüssigkeit abgelassen, d.h. das Volumen des Hohlkörpers verringert, erweitert sich der Innendurchmesser der Gehäuseeinbuchtung, sodass diese mit dem Impellermantel in Kontakt kommt, wodurch der Blutfluss in Richtung zum zweiten Ende des Gehäuses geleitet wird.

Gemäß einer zweiten Ausführungsform der Erfindung, ist die Blutpumpe bevorzugt derart weitergebildet, dass der Impeller in axialer Richtung des Gehäuses zwischen einer ersten und einer zweiten Position verschiebbar gelagert ist, wobei das Strömungsleitmittel in der ersten Position mit dem äußeren Rand der ringförmigen Öffnung und in der zweiten Position mit dem inneren Rand der ringförmigen Öffnung zusammenwirkt.

Das Strömungsleitmittel ist hierbei unbeweglich an der Innenseite des Gehäuses nahe der ringförmigen Öffnung angebracht und ragt in Richtung des Impellers radial in das Innere des Gehäuses vor. Um das Blut in Richtung des ersten axialen Endes bzw. des zweiten axialen Endes zu leiten, ist der Impeller axial in Richtung des ersten bzw. des zweiten axialen Endes des Gehäuses verschiebbar angeordnet.

Wird der Impeller in Richtung des ersten axialen Endes des Gehäuses verschoben, liegt das Strömungsleitmittel am Impellerkörper an, d.h. das Strömungsleitmittel wirkt mit dem inneren Rand der ringförmigen Öffnung zusammen, wodurch das Blut in Richtung des Herzens geleitet wird.

Wird der Impeller in Richtung des zweiten axialen Endes des Gehäuses verschoben, liegt das Strömungsleitmittel am Impellermantel an, d.h. das Strömungsleitmittel wirkt mit dem äußeren Rand der ringförmigen Öffnung zusammen, wodurch das Blut in Richtung der unteren Köperhälfte geleitet wird.

Erfindungsgemäß ist vorgesehen, dass der Ansaugschlauch der Pumpe ansaugseitig mit dem Impellermantel verbunden ist. Um die auf den Ansaugschlauch wirkende Zugkraft zu verringern und ein Verschieben des Impellers relativ zum Ansaugschlauch zu ermöglichen, ist bevorzugt vorgesehen, dass der Ansaugschlauch und der Impellermantel in axialer Richtung des Gehäuses verschiebbar miteinander verbunden sind.

Beispielweise kann der Ansaugschlauch hierfür mit einer Teleskopverbindung versehen sein, die in axialer Richtung der Pumpe verlängerbar ist, wodurch das Verschieben des Impellers in Richtung des zweiten axialen Endes der Pumpe unterstützt wird und die nach dem Verschieben auf den Ansaugschlauch wirkende Zugkraft wesentlich verringert wird.

Um eine axiale Verschiebung des Impellers in Richtung des zweiten axialen Endes der Pumpe zu ermöglichen, kann der Ansaugschlauch alternativ auch federnd gelagert sein.

Die Teleskopverbindung ist bevorzugt distal des ersten axialen Endes des Gehäuses, in unmittelbarer Nähe zum sich erweiternden Ende des Impellermantels angeordnet.

Um die flexible Antriebswelle im Gehäuse der Blutpumpe zu fixieren und zu stabilisieren, ist diese bevorzugt zu beiden Seiten des Impellers jeweils in einem Lager drehbar gehalten, das mittels einer radialen Verstrebung am Gehäuse befestigt ist. Die radiale Verstrebung kann hierbei wahlweise starr oder axial-federnd ausgebildet sein.

Die radiale Versstrebung erstreckt sich von der der Innenseite des Gehäuses zugerichteten Außenseite des Lagers in Richtung der Innenseite des Gehäuses, sodass die radiale Verstrebung an der Innenseite des Gehäuses zu liegen kommt. Hierdurch wird eine Abstützung der Antriebswelle am Gehäuse erreicht, wo die radiale Verstrebung in einer im Längsschnitt U-förmigen, ringförmigen Ausbuchtung des Gehäuses gehalten werden kann.

Bevorzugt kann das Lager als Fluid- oder Magnetlager ausgebildet sein.

Um die axiale Verschiebung des Impellers zu erleichtern, ist bevorzugt vorgesehen, dass das Gehäuse zu beiden Seiten der jeweiligen radialen Verstrebung jeweils eine in axialer Richtung komprimierbare und expandierbare Zone aufweist, welche eine axiale und federnde Verlagerung der radialen Verstrebung samt dem zugehörigen Lager erlaubt.

Die komprimierbaren und expandierbaren Zonen können beispielsweise als die Außenseite des Gehäuses umfangende Einbuchtungen oder Einschnürungen ausgebildet sein, die zu beiden Seiten der durch die radiale Verstrebung bedingten Ausbuchtung angeordnet sind.

Um den Wechsel der Ausstoßrichtung der Blutpumpe mit dem Herzschlag zu synchronisieren, ist bevorzugt vorgesehen, dass die Blutpumpe eine Steuervorrichtung umfasst, der EKG-Signale zuführbar sind und die mit einer Strömungsleitmittelbetätigung zusammenwirkt, um das austretende Blut in Abhängigkeit der EKG-Signale entweder zum ersten oder zweiten axialen Ende des Gehäuses zu leiten. Insbesondere kann die Steuervorrichtung eingerichtet sein, um eine intraaortale Gegenpulsation vorzunehmen. Dabei wird das Strömungsleitmittel während der aus den EKG-Signalen ermittelten Diastole in Richtung des zweiten Endes des Gehäuses geleitet und während der aus den EKG-Signalen ermittelten Systole in Richtung des ersten Endes des Gehäuses geleitet. Bevorzugt erfolgt die Umstellung hierbei bei jedem Herzschlag.

Alternativ kann vorgesehen sein, dass die Pumpe, **z.B. in** Abhängigkeit einer Sauerstoffsättigung des Blutes, über einen längeren Zeitraum, d.h. über mehrere Herzschläge hinweg, ohne Richtungsänderung betrieben wird. Hierdurch wird das Blut vermehrt in Richtung der oberen Körperhälfte oder in Richtung der unteren Köperhälfte gepumpt.

Um das Thromboserisiko zu verringern und ein optimiertes Strömungsverhalten des Blutes zu ermöglichen, ist bevorzugt vorgesehen, dass der Impellerkörper als flexibler Hohlkörper ausgebildet ist, der mit einer Gaszu- oder Flüssigkeitszu- und -abführungsleitung verbunden ist, um den Durchmesser des Impellerkörpers in gesteuerter Weise zu erhöhen oder zu verringern. Wenn der Impellerkörper hierbei bevorzugt eine Kegelfläche aufweist, die den Strömungskanal innen begrenzt, bewirkt ein Aufblasen bzw. Befüllen des Impellerkörpers eine Vergrößerung des Kegelwinkels, was eine steilere Anströmfläche erzeugt. Wird Gas oder Flüssigkeit aus dem Hohlkörper abgeführt, verringert sich der Öffnungswinkel des Kegels, sodass Blut in einem flacheren Strömungswinkel entlang des Impellerkörpers fließt. Ein geringer Kegelwinkel führt zu einer Verringerung negativer mechanischer Einflüsse auf Zellbestandteile des Blutes.

Die Änderung der Durchmessers des Impellerkörpers ermöglicht zusätzlich eine Veränderung des Volumenstroms, der in Richtung der oberen bzw. unteren Körperhälfte gepumpt wird. Eine Vergrößerung des Durchmessers des Impellerkörpers führt hierbei bei gleichbleibender Impellerdrehzahl zu einer Verringerung des gepumpten Blutvolumens je Zeiteinheit. Eine Verringerung des Durchmessers des Impellerkörpers führt bei gleichbleibender Impellerdrehzahl zu einer Erhöhung des gepumpten Blutvolumens je Zeiteinheit.

Bevorzugt ist hierbei vorgesehen, dass die Blutpumpe eine Steuervorrichtung umfasst, der Sensor- oder EKG-Signale zuführbar sind und die ausgebildet ist, um den Durchmesser des Impellerkörpers in Abhängigkeit der Sensor- oder EKG-Signale zu erhöhen oder zu verringern.

Zeigt das EKG beispielsweise eine ST-Hebung, was auf eine Ischämie hindeutet, wird der Durchmesser des Impellerköpers verringert, wodurch ein vermehrter Blutfluss in Richtung der oberen und unteren Körperhälfte ermöglicht und die Mangeldurchblutung behoben wird.

Die Sensorsignale können Messwerte eines physiologischen Parameters darstellen, die für den Durchblutungsgrad eines menschlichen Körpers oder einer Körperregion repräsentativ sind.

Bevorzugt ist vorgesehen, dass der flexible Hohlkörper aus Silikon, PU, Polyamid, PET oder PEBAX ausgebildet ist.

Bevorzugt kann der flexiblen Hohlkörper mit einem Nitinolgerüst versehen sein, um eine gleichmäße Expansion des Hohlkörpers zu gewährleisten.

Die erfindungsgemäße Blutpumpe wird nachfolgend anhand von in der Zeichnung schematisch dargestellten Ausführungsbeispielen näher erläutert. In dieser zeigen Fig.1 die erfindungsgemäße Blutpumpe gemäß einer ersten Ausführungsform der Erfindung, Fig.2 die erfindungsgemäße Blutpumpe gemäß einer zweiten Ausführungsform der Erfindung, Fig.3 eine Detailansicht der ersten Ausführungsform der Erfindung mit einem von einer ringförmigen, flexiblen Lippe gebildeten Strömungsleitmittel und Fig.4 eine Detailansicht der zweiten Ausführungsform der Erfindung.

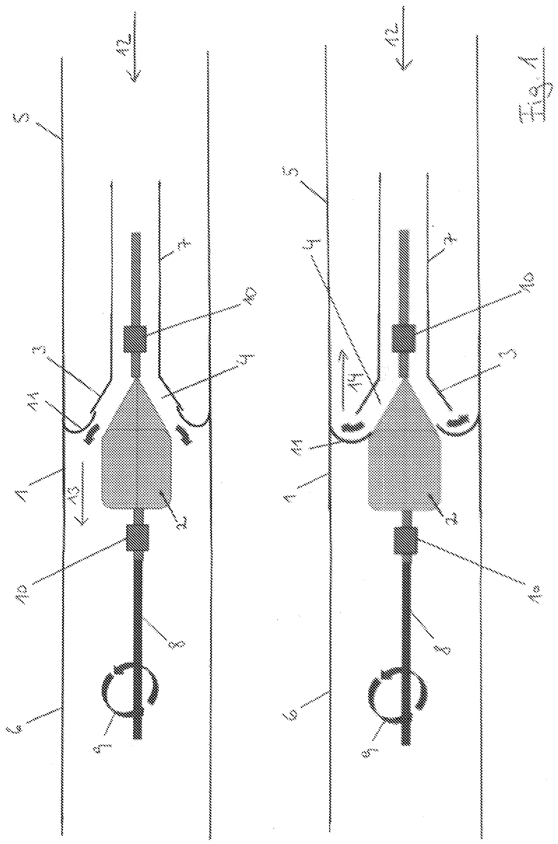

In Fig.1 ist die erfindungsgemäße Blutpumpe sowohl während der Systole (Fig.1 oben), als auch während der Diastole gezeigt (Fig.1 unten). Das Gehäuse der Blutpumpe ist mit 1 bezeichnet. Innerhalb des Gehäuses 1 ist ein drehbar gelagerter Impeller angeordnet, der einen inneren Impellerkörper 2 und einen Impellermantel 3 umfasst. Zwischen dem Impellerkörper 2 und dem Impellermantel 3 ist ein ringförmiger Strömungskanal 4 ausgebildet, der sich von einer dem ersten axialen Ende 5 des Gehäuses 1 zugewandten Ansaugseite zu einer dem zweiten axialen Endes 6 des Gehäuses 1 zugewandten Ausstoßseite erstreckt und sich radial nach außen erweitert. Der Impellermantel 3 ist mit einem Ansaugschlauch 7 verbunden, durch welchen angesaugtes Blut zum Strömungskanal 4 geleitet wird.

Der Impellerkörper 2 ist mit Hilfe einer flexiblen Antriebswelle 8 zu einer Drehbewegung antreibbar. Die Drehbewegung wird durch den mit 9 bezeichneten Pfeil angedeutet. Die Antriebswelle 8 ist zu beiden Seiten des Impellerkörpers 2 in einem Lager 10 gehalten.

Wird der Impellerkörper 2 in Drehung versetzt, erzeugen die am kegelförmigen Bereich des Impellerkörpers 2 angeordneten Impellerschaufeln (nicht dargestellt) einen Blutfluss, wobei Blut in Richtung des Pfeils 12 durch den Ansaugschlauch 7 in den zwischen Impellermantel 3 und Impellerkörper 2 befindlichen Strömungskanal 4 angesaugt wird. An der ringförmigen Öffnung des Strömungskanals 4, die außen vom Rand des Impellermantels 3 und innen vom Impellerkörper 2 begrenzt wird, ist ein vom Gehäuse 1 nach innen ragendes Strömungsleitmittel 11 angeordnet. Das Strömungsleitmittel ist im dargestellten Beispiel als ringförmige Lippe ausgebildet, die mittels eines nicht dargestellten Betätigungselements zwischen einer in Fig.1 oben dargestellten ersten Position und einer in Fig.1 unten dargestellten zweiten Position verlagerbar ist.

In Fig.1 oben ist das Strömungsleitmittel 11 derart ausgerichtet, dass es am Impellermantel 3 anliegt, wodurch das Blut aus der ringförmigen Öffnung in Richtung des Pfeils 13 strömt. In Fig.1 unten liegt das Strömungsleitmittel 11 am Impellerkörper 2 an, wodurch das aus der ringförmigen Öffnung kommende Blut in Richtung des Pfeils 14 umgeleitet wird und zum ersten axialen Ende 5 des Gehäuses 1 fließt.

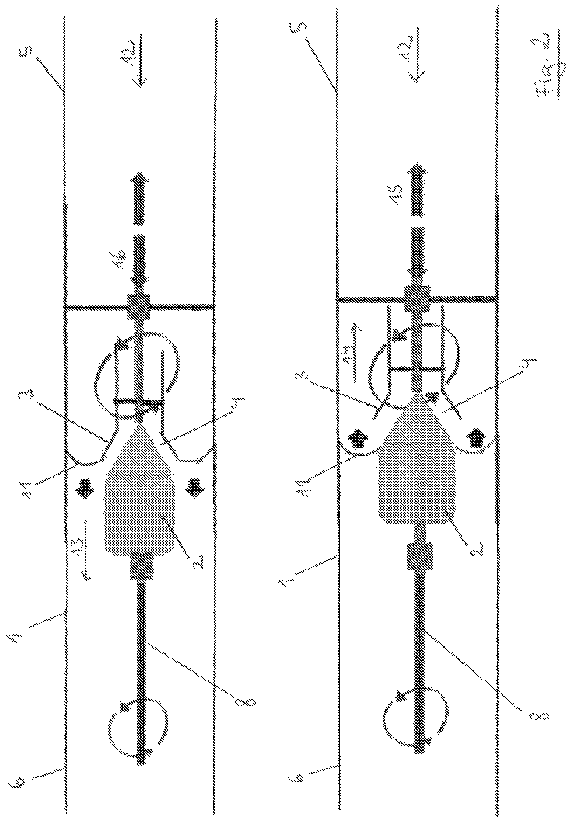

Fig.2 oben zeigt die erfindungsgemäße Blutpumpe gemäß einer zweiten Ausführungsform der Erfindung während der Systole. Fig.2 unten zeigt die erfindungsgemäße Blutpumpe gemäß einer zweiten Ausführungsform der Erfindung während der Diastole. Die in Fig.2 dargestellte Ausführungsform entspricht im Wesentlichen der in Fig. 1 gezeigten Ausführungsform, weist jedoch die folgenden Unterschiede auf.

Das Strömungsleitmittel 11 ist unbeweglich an der Innenseite des Gehäuses 1 angebracht und der Impeller, d.h. der Impellerkörper 2 und der Impellermantel 3, sind in axialer Richtung verschiebbar innerhalb des Gehäuses 1 gelagert. Um das in das Gehäuse 1 der Pumpe in Richtung des Pfeils 12 einströmende Blut wahlweise in Richtung des ersten axialen Endes 5 oder des zweiten axialen Endes 6 des Gehäuses 1 zu leiten, wird der Impeller entweder entsprechend des Pfeils 15 in Richtung des ersten axialen Endes 5 oder entsprechend des Pfeils 16 in Richtung des zweiten axialen Endes 6 bewegt.

Werden der Impellerkörper 2 und der Impellermantel 3 entsprechend des Pfeils 16 in Richtung des zweiten axialen Endes 6 des Gehäuses 1 verschoben, kommt das Strömungsleitmittel 11 am Impellermantel 3 zu liegen, wodurch das Blut in Richtung des Pfeils 13 strömt (Fig.2 oben). Werden der Impellerkörper 2 und der Impellermantel 3 hingegen entsprechend des Pfeils 15 in Richtung des ersten axialen Endes 5 des Gehäuses 1 verschoben, liegt das Strömungsleitmittel 11 am Impellerkörper 2 an, wodurch das Blut vom Strömungsleitmittel 11 in Richtung des Pfeils 14 zum ersten axialen Ende 5 des Gehäuses 1 strömt (Fig.2 unten).

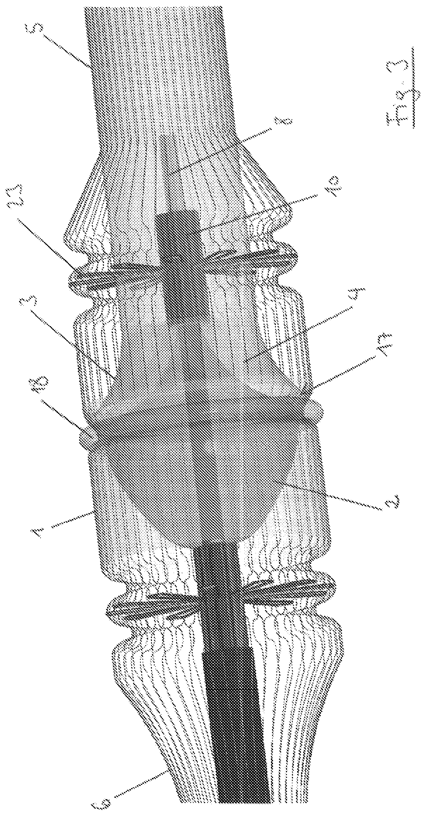

Fig.3 zeigt eine Detailansicht der ersten Ausführungsform der Erfindung, wobei das Strömungsleitmittel von einer ringförmigen Einschnürung 17 des Gehäuses 1 gebildet ist. Die Einschnürung 17 wirkt mit einem Betätigungsglied 18 zusammen, das als ringförmiger aufblasbarer, in der Einschnürung 17 liegender Hohlkörper ausgebildet ist.

Um einen mit Pfeil 14 gekennzeichneten Blutfluss in Richtung des ersten axialen Endes 5 des Gehäuses 1 zu bewirken, wird der Hohlkörper 18 mit Gas oder Flüssigkeit befüllt, wodurch sich die Einschnürung 17 verengt und mit dem inneren Rand der ringförmigen Öffnung des Strömungskanals 4, d.h. mit dem Impellerkörper 2, in Kontakt kommt, wodurch der Blutfluss in Richtung des ersten axialen Endes 5 des Gehäuses umgeleitet wird. Wird Gas oder Flüssigkeit aus dem Hohlkörper 18 abgeführt, vergrößert sich der Innendurchmesser der Einschnürung 17 wieder, sodass diese mit dem Impellermantel 3 in Kontakt kommt und einen Blutfluss in Richtung des zweiten axialen Endes 6 des Gehäuses 1 freigibt.

Um die Antriebswelle 8 im Gehäuse 1 der Blutpumpe zu fixieren, ist diese zu beiden Seiten des Impellers jeweils in einem Lager 10 drehbar gehalten, wobei das Lager 10 mittels einer radialen Verstrebung 23 an der Innenseite des Gehäuses 1 abgestützt ist.

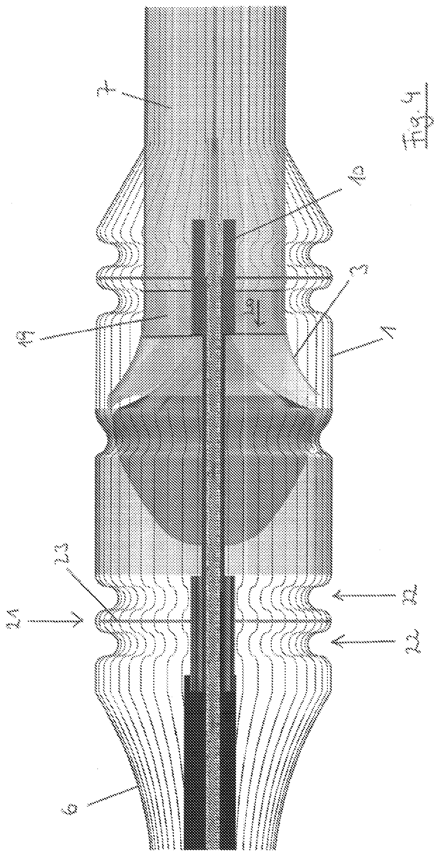

Fig.4 zeigt eine Detailansicht der zweiten Ausführungsform der Erfindung, d.h. jener Ausführungsform, bei welcher der Impeller axial verschiebbar im Gehäuse 1 der Pumpe aufgenommen ist. Der Impellermantel 3 ist über eine Teleskopverbindung 19 mit dem Ansaugschlauch 7 verbunden, die in axialer Richtung der Pumpe verlängerbar ist. Bei einer in Richtung des zweiten axialen Endes 6 des Gehäuses 1 gerichteten Bewegung des Impellermantels 3, verlängert sich die Teleskopverbindung 19 in Richtung des Pfeils 20, wodurch die auf den Ansaugschlauch 7 wirkende Zugkraft verringert und das Verschieben des Impellermantels 3 relativ zum Ansaugschlauch 7 vereinfacht wird.

Die in Fig.4 gezeigte Pumpe umfasst eine radiale Verstrebung 18, die sich vom Lager 10 in Richtung der Innenseite des Gehäuses 1 erstreckt, wo die Verstrebung 23 in einer ringförmigen Ausbuchtung 21 gehalten wird. Um eine federnde Translationsbewegung der Verstrebung 23 zu erlauben, weist das Gehäuse 1 zu beiden Seiten der Ausbuchtung 21 in axialer Richtung komprimierbare und expandierbare Zonen 22 auf, die jeweils von einer ringförmigen Einschnürung gebildet sind.

## Patentansprüche

1. Blutpumpe zur Unterstützung der Leistung eines Herzens, umfassend ein stentartiges Gehäuse (1), das ein erstes axiales Ende (5) und ein zweites axiales Ende (6)aufweist, einen im Gehäuse drehbar gelagerten Pumpenrotor, insbesondere Impeller, und eine flexible Antriebswelle (8), die mit einem Motor koppelbar ist, um den Pumpenrotor zur Drehbewegung anzutreiben, wodurch Blut auf einer Ansaugseite des Pumpenrotors durch einen Ansaugschlauch (7) der Blutpumpe eingesaugt und auf einer Ausstoßseite des Pumpenrotors ausgeworfen wird, **dadurch gekennzeichnet, dass** die Blutpumpe ein stromabwärts des Pumpenrotors angeordnetes Strömungsleitmittel (11) aufweist, um das vom Pumpenrotor geförderte Blut unter Beibehaltung der Drehrichtung des Pumpenrotors wahlweise zum ersten (5) oder zum zweiten axialen Ende (6) des Gehäuses (1) zu leiten.

2. Blutpumpe nach Anspruch 1, **dadurch gekennzeichnet, dass** der Impeller einen inneren Impellerkörper (2) aufweist, der eine Mehrzahl von Impellerschaufeln trägt und von einem im Wesentlichen konischen Impellermantel (3) derart umgeben ist, dass zwischen dem Impellermantel (3) und dem Impellerkörper (2) ein ringförmiger, sich in Strömungsrichtung von der Ansaugseite zu der Ausstoßseite radial nach außen erweiternder Strömungskanal (4) ausgebildet wird, in dem die Impellerschaufeln angeordnet sind, wobei ansaugseitig der Ansaugschlauch mit dem Impellermantel (3) verbunden ist und der Strömungskanal (4) ausstoßseitig eine ringförmige Öffnung aufweist, deren äußerer Rand von dem Impellermantel (3) und deren innerer Rand von dem Impellerkörper (2) gebildet ist, und wobei das Gehäuse (1) das nach innen ragende Strömungsleitmittel (11) aufweist, das wahlweise mit dem inneren oder dem äußeren Rand der ringförmigen Öffnung zusammenwirkt, um das aus dem Strömungskanal (4) des Impellers austretende Blut entweder zum ersten (5) oder zum zweiten axialen Ende (6) des Gehäuses (1) zu leiten.

3. Blutpumpe nach Anspruch 2, **dadurch gekennzeichnet, dass** das Strömungsleitmittel (11) zwischen einer ersten und einer zweiten Position verlagerbar gehalten ist, wobei das Strömungsleitmittel (11) in der ersten Position mit dem äußeren Rand der ringförmigen Öffnung und in der zweiten Position mit dem inneren Rand der ringförmigen Öffnung zusammenwirkt.

4. Blutpumpe nach Anspruch 3, **dadurch gekennzeichnet, dass** das Strömungsleitmittel (11) von einer ringförmigen, flexiblen Lippe gebildet ist und dass ein vorzugsweise pneumatisch oder hydraulisch betätigbares Betätigungsglied (18) vorgesehen ist, welches mit dem Strömungsleitmittel (11) zusammenwirkt, um dieses zwischen der ersten und der zweiten Position zu verlagern.

5. Blutpumpe nach Anspruch 4, **dadurch gekennzeichnet, dass** das pneumatisch oder hydraulisch betätigbare Betätigungsglied (18) von einem ringförmigen Hohlkörper (18) gebildet ist, der mit einer Gaszu- oder Flüssigkeitszu- und -abführungsleitung verbunden ist, um den Füllgrad des Hohlkörpers (18) in gesteuerter Weise zu erhöhen oder zu verringern.

6. Blutpumpe nach Anspruch 2, **dadurch gekennzeichnet, dass** der Impeller in axialer Richtung des Gehäuses (1) zwischen einer ersten und einer zweiten Position verschiebbar gelagert ist, wobei das Strömungsleitmittel (11) in der ersten Position mit dem äußeren Rand der ringförmigen Öffnung und in der zweiten Position mit dem inneren Rand der ringförmigen Öffnung zusammenwirkt.

7. Blutpumpe nach Anspruch 5, **dadurch gekennzeichnet, dass** der Ansaugschlauch (7) und der Impellermantel (3) in axialer Richtung des Gehäuses (1) verschiebbar miteinander verbunden sind.

8. Blutpumpe nach einem der Ansprüche 1 bis 7, **dadurch gekennzeichnet, dass** die flexible Antriebswelle (8) zu beiden Seiten des Pumpenrotors, insbesondere Impellers, jeweils in einem Lager (10) drehbar gehalten ist, das mittels einer radialen Verstrebung (23) am Gehäuse (1) befestigt ist.

9. Blutpumpe nach Anspruch 8, **dadurch gekennzeichnet, dass** das Gehäuse (1) zu beiden Seiten der jeweiligen radialen Verstrebung (23) jeweils eine in axialer Richtung komprimierbare und expandierbare Zone (22) aufweist, welche eine axiale und federnde Verlagerung der radialen Verstrebung (23) samt dem zugehörigen Lager (10) erlaubt.

10. Blutpumpe nach einem der Ansprüche 1 bis 9, **dadurch gekennzeichnet, dass** die Blutpumpe eine Steuervorrichtung umfasst, der EKG-Signale zuführbar sind und die mit einer Strömungsleitmittelbetätigung zusammenwirkt, um das austretende Blut in Abhängigkeit der EKG-Signale entweder zum ersten (5) oder zweiten axialen Ende (6) des Gehäuses (1) zu leiten.

11. Blutpumpe nach einem der Ansprüche 2 bis 10, **dadurch gekennzeichnet, dass** der Impellerkörper (2) als flexibler Hohlkörper ausgebildet, der der mit einer Gaszu- oder Flüssigkeitszu- und -abführungsleitung verbunden ist, um den Durchmesser des Impellerkörpers (2) in gesteuerter Weise zu erhöhen oder zu verringern.

12. Blutpumpe nach Anspruch 11, **dadurch gekennzeichnet, dass** die Blutpumpe eine Steuervorrichtung umfasst, der Sensor- oder EKG-Signale zuführbar sind und die ausgebildet ist, um den Durchmesser des Impellerkörpers (2) in Abhängigkeit der Sensor- oder EKG-Signale zu erhöhen oder zu verringern.

## Claims

1. A blood pump for supporting cardiac performance, comprising a stent-like housing (1) which has a first axial end (5) and a second axial end (6), a pump rotor mounted rotatably in the housing, in particular an impeller, and a flexible drive shaft (8), which can be coupled to a motor in order to drive the pump rotor to rotate, whereby blood is sucked in on an intake side of the pump rotor through an intake tube (7) of the blood pump and is ejected on a discharge side of the pump rotor, **characterized in that** the blood pump comprises flow directing means (11) arranged downstream of the pump rotor in order to selectively direct the blood conveyed by the pump rotor to the first (5) or to the second axial end (6) of the housing (1) whilst maintaining the direction of rotation of the pump rotor.

2. The blood pump according to claim 1, **characterized in that** the impeller comprises an inner impeller body (2), which carries a multitude of impeller blades and is surrounded by a substantially conical impeller casing (3) in such a way that, between the impeller casing (3) and the impeller body (2), an annular flow channel (4) is formed which widens radially outwards in the direction of flow from the intake side to the discharge side, in which the impeller blades are arranged, wherein the intake tube is connected to the impeller casing (3) on the intake side and the flow channel (4) has an annular opening on the discharge side, the outer edge of which is formed by the impeller casing (3) and the inner edge of which is formed by the impeller body (2), and wherein the housing (1) comprises the inwardly protruding flow directing means (11), which interacts selectively with the inner or the outer edge of the annular opening, in order to guide the blood emerging from the flow channel (4) of the impeller either to the first (5) or to the second (6) axial end of the housing (1).

3. The blood pump according to claim 2, **characterized in that** the flow directing means (11) is displaceably supported between a first and a second position, the flow directing means (11) interacting with the outer edge of the annular opening in the first position and with the inner edge of the annular opening in the second position.

4. The blood pump according to claim 3, **characterized in that** the flow directing means (11) is formed by an annular, flexible lip, and **in that** a preferably pneumatically or hydraulically actuatable actuating member (18) is provided, which interacts with the flow directing means (11) in order to displace the latter between the first and the second position.

5. The blood pump according to claim 4, **characterized in that** the pneumatically or hydraulically actuatable actuating member (18) is formed by an annular hollow body (18) which is connected to a gas supply or liquid supply and discharge line in order to increase or decrease the degree of filling of the hollow body (18) in a controlled manner.

6. The blood pump according to claim 2, **characterized in that** the impeller is slidably supported in the axial direction of the housing (1) between a first and a second position, wherein the flow directing means (11) interacts with the outer edge of the annular opening in the first position and with the inner edge of the annular opening in the second position.

7. The blood pump according to claim 5, **characterized in that** the intake tube (7) and the impeller casing (3) are connected to each other so as to be displaceable in the axial direction of the housing (1).

8. The blood pump according to any one of claims 1 to 7, **characterized in that** the flexible drive shaft (8) is rotatably held on both sides of the pump rotor, in particular impeller, in a bearing (10) that is fastened to the housing (1) by means of a radial strutting (23).

9. The blood pump according to claim 8, **characterized in that** the housing (1) has an axially compressible and expandable zone (22) on both sides of the respective radial strutting (23), which allows an axial and resilient displacement of the radial strutting (23) together with the associated bearing (10).

10. The blood pump according to any one of claims 1 to 9, **characterized in that** the blood pump comprises a control device to which ECG signals can be supplied and which cooperates with a flow directing means actuator in order to guide the ejected blood to either the first (5) or second (6) axial end of the housing (1) depending on the ECG signals.

11. The blood pump according to any one of claims 2 to 10, **characterized in that** the impeller body (2) is configured as a flexible hollow body that is connected to a gas or liquid supply and discharge line in order to increase or decrease the diameter of the impeller body (2) in a controlled manner.

12. The blood pump according to claim 11, **characterized in that** the blood pump comprises a control device to which sensor or ECG signals can be supplied and which is configured to increase or decrease the diameter of the impeller body (2) depending on the sensor or ECG signals.

## Revendications

1. Pompe à sang pour soutenir la performance d'un cœur, comprenant un boîtier de type stent (1) qui présente une première extrémité axiale (5) et une seconde extrémité axiale (6), un rotor de pompe, en particulier une roue ailée, monté de manière rotative dans le boîtier, et un arbre d'entraînement flexible (8) qui peut être couplé à un moteur pour entraîner le rotor de pompe en mouvement rotatif, de sorte que le sang est aspiré par un tuyau d'aspiration (7) de la pompe à sang du côté d'aspiration du rotor de pompe et éjecté du côté d'éjection du rotor de pompe, **caractérisée en ce que** la pompe à sang comprend un moyen de guidage d'écoulement (11) disposé en aval du rotor de pompe pour diriger le sang pompé par le rotor de pompe, tout en maintenant la direction de rotation du rotor de pompe, sélectivement vers la première (5) ou la seconde extrémité axiale (6) du boîtier (1).

2. Pompe à sang selon la revendication 1, **caractérisée en ce que** la roue ailée comprend un corps de roue ailée interne (2) qui porte une pluralité d'aubes de roue ailée et qui est entouré par une enveloppe de roue ailée essentiellement conique (3) de telle sorte qu'un canal d'écoulement annulaire (4) s'élargissant radialement vers l'extérieur dans la direction d'écoulement du côté d'aspiration au côté d'éjection est formé entre l'enveloppe de roue ailée (3) et le corps de roue ailée (2), dans lequel les aubes de roue ailée sont disposées, le tuyau d'aspiration étant relié à l'enveloppe de roue ailée (3) du côté d'aspiration et le canal d'écoulement (4) présentant une ouverture annulaire du côté d'éjection, dont le bord extérieur est formé par l'enveloppe de roue ailée (3) et le bord intérieur par le corps de roue ailée (2), et où le boîtier (1) comprend le moyen de guidage d'écoulement (11) faisant saillie vers l'intérieur, qui coopère sélectivement avec le bord intérieur ou extérieur de l'ouverture annulaire pour diriger le sang sortant du canal d'écoulement (4) de la roue ailée soit vers la première (5) soit vers la seconde extrémité axiale (6) du boîtier (1).

3. Pompe à sang selon la revendication 2, **caractérisée en ce que** le moyen de guidage d'écoulement (11) est monté de manière déplaçable entre une première et une seconde position, le moyen de guidage d'écoulement (11) coopérant dans la première position avec le bord extérieur de l'ouverture annulaire et dans la seconde position avec le bord intérieur de l'ouverture annulaire.

4. Pompe à sang selon la revendication 3, **caractérisée en ce que** le moyen de guidage d'écoulement (11) est formé par une lèvre annulaire flexible et qu'un élément d'actionnement (18), de préférence actionné pneumatiquement ou hydrauliquement, est prévu, qui coopère avec le moyen de guidage d'écoulement (11) pour le déplacer entre la première et la seconde position.

5. Pompe à sang selon la revendication 4, **caractérisée en ce que** l'élément d'actionnement pneumatique ou hydraulique (18) est formé par un corps creux annulaire (18) qui est relié à une conduite d'alimentation et d'évacuation de gaz ou de liquide pour augmenter ou diminuer de manière contrôlée le niveau de remplissage du corps creux (18).

6. Pompe à sang selon la revendication 2, **caractérisée en ce que** la roue ailée est monté de manière déplaçable dans la direction axiale du boîtier (1) entre une première et une seconde position, le moyen de guidage d'écoulement (11) coopérant dans la première position avec le bord extérieur de l'ouverture annulaire et dans la seconde position avec le bord intérieur de l'ouverture annulaire.

7. Pompe à sang selon la revendication 5, **caractérisée en ce que** le tuyau d'aspiration (7) et l'enveloppe de roue ailée (3) sont reliés de manière déplaçable dans la direction axiale du boîtier (1).

8. Pompe à sang selon l'une des revendications 1 à 7, **caractérisée en ce que** l'arbre d'entraînement flexible (8) est maintenu en rotation des deux côtés du rotor de pompe, en particulier de la roue ailée, dans un palier (10) qui est fixé au boîtier (1) au moyen d'une entretoise radiale (23).

9. Pompe à sang selon la revendication 8, **caractérisée en ce que** le boîtier (1) présente, des deux côtés de l'entretoise radiale respective (23), une zone (22) compressible et expansible dans la direction axiale, qui permet un déplacement axial et élastique de l'entretoise radiale (23) avec le palier associé (10).

10. Pompe à sang selon l'une des revendications 1 à 9, **caractérisée en ce que** la pompe à sang comprend un dispositif de commande auquel des signaux ECG peuvent être fournis et qui coopère avec un actionnement du moyen de guidage d'écoulement pour diriger le sang sortant soit vers la première (5) soit vers la seconde extrémité axiale (6) du boîtier (1) en fonction des signaux ECG.

11. Pompe à sang selon l'une des revendications 2 à 10, **caractérisée en ce que** le corps de roue ailée (2) est conçu comme un corps creux flexible qui est relié à une conduite d'alimentation et d'évacuation de gaz ou de liquide pour augmenter ou diminuer de manière contrôlée le diamètre du corps de roue ailée (2).

12. Pompe à sang selon la revendication 11, **caractérisée en ce que** la pompe à sang comprend un dispositif de commande auquel des signaux de capteur ou des signaux ECG peuvent être fournis et qui est conçu pour augmenter ou diminuer le diamètre du corps de roue ailée (2) en fonction des signaux de capteur ou des signaux ECG.
